# FASCICULE DE BREVET EUROPEEN

(11) **EP 1 135 168 B1**
(45) Date de publication et mention de la délivrance du brevet: **20.04.2005**
(21) Numéro de dépôt: 99972931.2
(22) Date de dépôt: 26.11.1999
(51) Int. Cl.: A61K 47/48

(54) **VECTEURS PEPTIDIQUES DE SUBSTANCES A TRAVERS LA BARRIERE HEMATO-ENCEPHALIQUE**
PEPTIDVEKTOREN VON STOFFEN DIE DURCH DIE BLUT-HIRN-SCRANKE DRINGEN
PEPTIDES CARRYING SUBSTANCES ACROSS THE BLOOD BRAIN BARRIER

(30) Priorité: 30.11.1998 FR 9815074
(43) Date de publication de la demande: 26.09.2001
(73) Titulaire: Synt:em (S.A.), 30000 Nîmes (FR)
(72) Inventeur: CLAIR, Philippe, F-30000 Nîmes (FR); KACZOREK, Michel, F-34980 Montferrier sur Lez (FR); TEMSAMANI, Jamal Résidence de l'Empereur, F-30900 Nîmes (FR)
(74) Mandataire: Breese, Pierre
(86) Numéro de dépôt international: PCT/FR1999/002938
(87) Numéro de publication internationale: WO 2000/032236

(56) Documents cités:
- WO-A-00/29427
- WO-A-97/12912
- WO-A-97/19954
- WO-A-98/46250
- WO-A-99/07728
- DEROSSI D. ET AL: "The third helix of the Antennapedia homeodomain translocates through biological membranes." JOURNAL OF BIOLOGICAL CHEMISTRY., vol. 269, no. 14, 1994, pages 10444-10450, XP002114617 AMERICAN SOCIETY OF BIOLOGICAL CHEMISTS, BALTIMORE, MD., US ISSN: 0021-9258 cité dans la demande

## Description

La présente invention concerne l'utilisation des peptides comme vecteurs pour le transfert de molécules actives à travers la barrière hémato-encéphalique (BHE) pour des applications en thérapie et en diagnostic.

Le problème majeur dans le traitement de nombreuses maladies du système nerveux central réside dans le fait que les molécules administrées ne passent pas la barrière hémato-encéphalique et ne peuvent donc pas atteindre leur cible dans le cerveau.

Les cellules endothéliales qui constituent la BHE font obstacle, de diverses manières, aux molécules qui tentent de les franchir. En effet, ces cellules endothéliales constituent une barrière physique représentée par les jonctions étanches qui relient entre elles et empêchent tout passage par la voie paracellulaire et ce, d'autant plus que l'activité d'endocytose y est faible, ce qui limite fortement le passage des substances du plasma vers l'espace extracellulaire cérébral.

Une des priorités de la recherche dans ce domaine, est donc de trouver des moyens permettant d'augmenter l'efficacité de passage des substances actives à travers la BHE. Plusieurs stratégies ont été développés pour augmenter le passage de ces substances à travers la BHE (Pardridge, 1994, Tibtech 12, 239-245 ; Tamai et al., 1996, Adv. Drug Del. Rev. 19, 401-424).

Trois stratégies principales ont été proposées pour- le transport de molécules à travers la BHE, une stratégie neurochirurgicale, une stratégie pharmacologique pour les petites molécules et une stratégie physiologique.

La stratégie neurochirurgicale peut être mise en oeuvre par infusion intraventriculaire de la substance active, par thérapie cellulaire, et par perturbation de la BHE. L'infusion intraventriculaire implique le placement d'un cathéter dans les ventricules (Aird, 1984, Exp. Neurol. 86, 342-358). Cette technique est très invasive et n'est pas efficace pour le transport de substances actives dans le parenchyme. La perturbation de la BHE cause une ouverture transitoire des jonctions serrées, cas des substances vasoactives comme les leukotriènes ou bradykinines (Baba et al., 1991, J. Cereb. Blood Flow Metab. 11, 638-643). Cette stratégie est également invasive et nécessite un accès artériel chez les sujets sous sédatifs. En outre, la perturbation répétitive de la BHE peut conduire à des changements neuropathiques (Salahuddin et al., 1988, Acta Neuropathol. 76, 1-10).

La stratégie pharmacologique pour le transport de petites molécules comprend l'addition de groupements lipidiques et l'utilisation des liposomes (Zhou et al., 1992, J. Controlled Release 19, 459-486). L'addition d'un groupement lipidique permet la conversion chimique des molécules solubles dans l'eau en molécules solubles dans les lipides. Cependant, la synthèse de tels produits conduit à des molécules qui dépassent le seuil de transport. Les molécules doivent avoir un poids moléculaire de moins de 600d pour traverser la BHE. Pour cette raison, les liposomes ou même les petites vésicules sont trop grandes et par conséquent inefficaces pour le transport à travers la BHE (Levin, 1980, J. Med. Chem. 23, 682-684 ; Schackert et al., 1989, Selective Cancer Ther. 5, 73-79).

La stratégie physiologique fait appel à un système de transport récepteur-dépendent. La molécule à transporter est couplée à une molécule biologique qui possède un récepteur au niveau de la BHE. Par exemple, la transferrine possède un récepteur au niveau de la BHE et peut être utilisée comme vecteur (Jeffries et al., 1984, Nature 312, 162-163 ; Friden et al., 1983, Science 259, 373-377 ; Friden, 1994, Neurosurgery 35, 294-298). Bien que cette stratégie permette une augmentation du passage des molécules à travers la BHE, elle présente quelques inconvénients. D'abord, le couplage de la molécule au vecteur se fait par des méthodes d'expression génétique limitant ainsi le nombre de molécules à transporter à seulement des polypeptides ou des protéines. Ensuite, le système de couplage de la molécule au vecteur est compliqué.

La présente invention vise donc à palier ces inconvénients en utilisant des peptides pour vectoriser des substances à travers la barrière hémato-encéphalique (BHE). Cette approche offre plusieurs avantages. Tout d'abord, le peptide vecteur est synthétisé par voie chimique. De plus, la plupart des molécules médicamenteuses (molécules conventionnelles, peptides, protéines, oligonucléotides) peuvent être couplées au vecteur de façon simple et efficace.

Il a été décrit dans l'art antérieurs de nombreux peptides capables de traverser les membranes des cellules eucaryotes de manière très rapide et tels que les peptides suivants : Protégrine, Antennapedia, Tachyplésine, Transportan, etc....

Parmi ceux-ci, certains présentent des propriétés cytolytiques. Ces peptides dénommés peptides antibiotiques sont notamment les Protégrines et Tachyplésines. Les Protégrines et Tachyplésine sont des peptides antibiotiques naturels dont la structure est de type épingle à cheveux maintenue par des ponts disulfures. Ces ponts jouent un rôle important dans l'activité cytolytique observée sur cellules humaines.

Selon leur structure, les peptides antibiotiques peuvent être classés en trois grandes familles :
- Les- peptides antibiotiques à hélices alpha amphipatiques : cécropines et maganines (Maloy, W. L. et al., 1995, BioPolymer 37, 105-122).
- Les peptides antibiotiques à feuillets bêta réunis par des ponts disulfures : défensines (Lehrer, R. I. et al., 1991, Cell 64:229-230 ; Lehrer, R. I. et al., 1993, Ann. Rev. Immunol. 11:105-128), protégrines (Kokryakov, V. N. et al., 1993, FEBS 337:231-236), tachyplésines (Nakamura, T. et al., 1988, J. Biol. Chem. 263:16709-16713 ; Miyata, T et al., 1989, J. Biochem. 106:663-668).
- les peptides antibiotiques à chaînes déstructurées contenant de nombreux coudes liés à la présence de multiples prolines : bacténécines et PR39 (Frank, R. W. et al., 1991, Eur. J. Biochem. 202, 849-854).

On désigne sous le nom de protégrines un ensemble de cinq peptides désignés PG-1, PG-2, PG-3, PG-4 et PG-5 dont les séquences sont données ci-dessous, étroitement apparentés et isolés de leucocytes de porc (V.N. Kokryakov & col. FEBS lett. 327, 231-236) :
PG-1 : RGGRLCYCRRRFCVCVGR-NH₂
PG-2 : RGGRLCYCRRRFCICV..-NH₂
PG-3 : RGGGLCYCRRRFCVCVGR-NH₂
PG-4 : RGGRLCYCRGWICFCVGR-NH₂
PG-5 : RGGRLCYCRPRFCVCVGR-NH₂

Les tachyplésines (Tamura, H. et al., 1993, Chem. Pharm. Bul. Tokyo 41, 978-980), désignées T1, T2 et T3 et les polyphémusines (Muta, T., 1994, CIBA Found. Sym. 186, 160-174), désignées P1 et P2, dont les séquences sont données ci-dessous, sont des peptides homologues isolés de l'hémolymphe de deux crabes, *Tachyplesus tridentatus* pour les tachyplésines T1, T2 et T3 et *Limmulu's polyphemus* pour les polyphémusines P1 et P2 :
P1 : RRWCFRVCYRGFCYRKCR-NH₂
P2 : RRWCFRVCYKGFCYRKCR-NH₂

Protégrines, tachyplésines et polyphémusines contiennent une forte proportion de résidus basiques (lysines et arginines) et possédent quatre cystéines qui forment deux ponts disulfures paralléles. Ces trois familles de peptides présentent également des homologies avec certaines défensines et en particulier avec la défensine humaine NP-1 (Kokryakov, V. N. et al., 1993, Febs Let. 327, 231-236).

Ainsi, dans le cadre de ces travaux de recherche, la Demanderesse a découvert que la réduction irréversible de ces ponts disulfures permet d'obtenir des peptides linéaires, désignés ci-après aussi "Pégélines" ayant la capacité de traverser rapidement les membranes des cellules de mammifères par un mécanisme passif ne faisant pas appel à un récepteur membranaire. Ces peptides linéaires sont non-toxiques et sans activité lytique, et en conséquence, ils constituent un nouveau système de vectorisation de substances actives dans les domaines thérapeutiques ou de diagnostic. Les travaux et résultats concernant ces peptides linéaires et leur utilisation comme vecteur de substances actives sont décrits dans la demande de brevet français de la Demanderesse déposée le 12 Août 1998 sous le No. 97/10297.

Les peptides issus de la famille Antennapedia sont des dérivés du facteur de transcription de l'homéodomaine Antennapedia de la mouche drosophile et sont par exemple décrits dans les demandes de brevet internationales PCT publiées sous les No. WO91/18981 et WO97/12912. La séquence de ces peptides présente la particularité d'être hautement conservée dans toutes les homéoprotéines. Ces peptides sont composés de trois hélices alpha et sont capables de se transloquer au travers de la membrane cellulaire. Le plus petit fragment de l'homéodomaine capable de traverser les membranes est un peptide de 16 acides aminés (Prochiantz, 1996, Curr. Opin. In Neurob. 6, 629-634 ; Derossi et al., 1994, J. Biol. Chem. 269, 10444-10450).

Les travaux de recherche réalisés dans le cadre de la présente invention ont maintenant permis à la Demanderesse de montrer que certains de ces peptides linéaires, c'est-à-dire dépourvus de pont disulfure, peuvent être utilisés comme système de vectorisation très efficace permettant de faire traverser la BHE à une substance active en diagnostic ou thérapie d'une affection du système nerveux central (SNC).

L'invention concerne donc plus particulièrement l'utilisation d'un peptide linéaire couplé à une substance active en diagnostic ou thérapie d'une affection du SNC pour la préparation d'un médicament capable de traverser le barrière hématoencéphalique à utiliser en diagnostic ou thérapie d'une affection localisée au niveau du SNC, ledit peptide répondant à l'une des formules (I), (II) ou (III) suivantes :

X₁-X₂-X₃-X₄-X₅-X₆-X₇-X₈-X₉-X₁₀-X₁₁-X₁₂-X₁₃-X₁₄-X₁₅-X₁₆ (I)

dans laquelle formule (I), les résidus X₁ à X₁₆ sont des résidus d'acides aminés dont 6 à 10 d'entre-eux sont des acides aminés hydrophobes et X₆ est le tryptophane,

BXXBXXXXBBBXXXXXXB (II)

BXXXBXXXBXXXXBBXB (III),

dans lesquelles formules (II) et (III) :
- les groupes B, identiques ou différents, représentent un résidus d'acide aminé dont la chaîne latérale porte un groupement basique, et
- les groupes X, identiques ou différents, représentent un résidus d'acide aminé aliphatique ou aromatique,
ou lesdits peptides de formules (I), (II), (III) sous forme rétro, constitués d'acides aminés de configuration D et/ou L, ou un fragment de ceux-ci constitué d'une séquence d'au moins 5 et de préférence d'au moins 7 acides aminés successifs des peptides de formules (I), (II) ou (III) dès lors bien entendu que ce fragment présente les propriétés de vectorisation sans toxicité pour les cellules.

Les peptides de formule (I) dérivent de la famille Antennapedia. Dans les peptides de formules (I), les acides aminés hydrophobes sont l'alanine, la valine, la leucine, l'isoleucine, la proline, la phénylalanine, le tryptophane, la tyrosine et la méthionine, et les autres acides aminés sont des acides aminés :
- non-hydrophobes qui peuvent être des acides aminés non polaire comme la glycine, ou polaires comme la sérine, la thréonine, la cystéine, l'asparagine, la glutamine, ou
- acides (acide aspartique ou glutamique), ou
- basiques (lysine, arginine ou histidine), ou
- une association d'acides aminés de ces trois catégories.

Parmi les peptides de formule (I), on préfère ceux comprenant 6 acides aminés hydrophobes et 10 acides aminés non-hydrophobes.

Les peptides linéaires de formule (II) dérivent de la famille Protégrine et les peptides linéaires de formule (III) dérivent de la famille Tachyplésine. Parmi les peptides de formules (II) et (III), on préfère ceux dans lesquels :
- B est choisi parmi l'arginine, la lysine, l'acide diaminoacétique, l'acide diaminobutyrique, l'acide diaminopropionique, l'ornithine, et
- X est choisi parmi la glycine, l'alanine, la valine, la norleucine, l'isoleucine, la leucine, la cystéine, la cystéine^{Acm}, la penicillamine, la méthionine, le serine, la thréonine, l'asparagine, la glutamine, la phényalanine, l'histidine, le tryptophane, la tyrosine,. la proline, l'Abu, l'acide amino-1-cyclohexane carboxylique, l'Aib, la 2-aminotétraline carboxylique, la 4-bromophényalanine, tert-Leucine, la 4-chlorophénylalanine, la bêta-cyclohexyalanine, la 3,4-dichlorophényalanine, la 4-fluorophényalanine, l'homoleucine, la bêta-homoleucine, l'homophényalanine, la 4-méthylphényalanine, la 1-naphtyalanine, la 2- naphtyalanine, la 4-nitrophényalanine, la 3-nitrotyrosine, la norvaline, la phénylglycine, la 3-pyridyalanine, la [2-thiényl]alanine.

Dans les peptides de formules (I), (II) ou (III), B, X et X₁ à X₁₆ peuvent être des acides aminés naturels ou non, y compris des acides aminés de configuration D.

Des peptides préférés utilisés selon l'invention sont choisis parmi ceux dont les séquences en acides aminés sont les suivantes :
- RGGRLSYSRRRFSTSTGR, désigné aussi ci-après SynB 1
- RRLSYSRRRF, désigné aussi ci-après SynB 3
- rqikiwfqnrrmkwkk
où les lettres minuscules représentent des acides aminés sous forme d.

Le couplage de la substance active et d'un peptide défini ci-dessus dans les compositions de l'invention peut être réalisé par tout moyen de liaison acceptable compte tenu de la nature chimique, de l'encombrement et du nombre de substance active et de peptide associés. Il peut s'agir de liaisons covalentes, hydrophobes ou ioniques, clivables ou non-clivables dans les milieux physiologiques ou à l'intérieur de la cellules.

Le couplage peut être effectué en n'importe quel site du peptide, dans lequel des groupements fonctionnels tels que -OH, -SH, -COOH, -NH₂ sont naturellement présents ou ont été introduits. Ainsi, un agent anti-cancéreux peut être lié au peptide au niveau des extrémités N-terminale ou C-terminale ou bien au niveau des chaînes latérales du peptide.

De même, le couplage peut être effectué en n'importe quel site de la substance active, où par exemple des groupements fonctionnels tels que -OH, -SH, -COOH, -NH₂ sont naturellement présents ou ont été introduits.

Ainsi, l'invention se rapporte tout particulièrement à l'utilisation de composés répondant à la formule (IV) suivante :

A (-)ₘ (B)ₙ (IV)

dans laquelle
- A représente un peptide tel que défini précédemment,
- B représente une substance active en diagnostic ou thérapie d'une affection du SNC,
- n est 1 ou plus, et de préférence jusqu'à 10 avantageusement jusqu'à 5,
- (-)ₘ représente la liaison, ou linker, entre A et B, où m est 1 ou plus, et de préférence jusqu'à 10 avantageusement jusqu'à 5,
pour la préparation d'un médicament capable de traverser le barrière hémotoencéphalique à utiliser en diagnostic ou thérapie d'une affection localisée au niveau du SNC.

Dans la formule (IV) la liaison (-)ₘ entre A et B est une liaison covalente, hydrophobe ou ionique, clivable ou non-clivable dans les milieux physiologiques ou à l'intérieur de la cellules, ou un mélange de celles-ci.

Les composés de formule (IV) peuvent être préparés par synthèse chimique ou en utilisant des techniques de biologie moléculaire.

On peut utiliser pour les synthèses chimiques des appareils commerciaux permettant d'incorporer des acides aminés non-naturels, tels que les énantiomères D et des résidus ayant des chaînes latérales ayant des hydrophobicités et des encombrements différents de ceux de leurs homologues naturels. Au cours de la synthèse, il est évidemment possible de réaliser un large éventail de modifications, par exemple introduire sur le N-terminal un lipide, par exemple prenyl ou myristyl, de façon à pouvoir ancrer le peptide de l'invention et donc le composé de formule (IV) à une membrane lipidique telle que celle d'un liposome constitué de lipides. Il est également possible de remplacer une ou plusieurs liaisons peptidiques (-CO-NH-) par des structures équivalentes comme -CO-N(CH₃)-, -CH₂-CH₂-, -CO-CH₂-, ou bien d'intercaller des groupes comme -CH₂-, -NH-, -O-.

On peut également obtenir les composés de formule (IV) ou partie de ceux-ci de nature protéique à partir d'une séquence d'acide nucléique codant pour celui-ci. La présente invention a aussi pour objet une molécule d'acide nucléique comprenant ou constituée par une séquence nucléique codant pour un peptide linéaire dérivé de peptide antibiotique. Plus particulièrement l'invention concerne une molécule d'acide nucléique comprenant au moins une séquence codant pour un composé de formule (IV) ou une partie de celui de nature.protéique. Ces séquences d'acides nucléiques peuvent être des ADN ou ARN et être associées à des séquences de contrôle et/ou être insérées dans des vecteurs. Le vecteur utilisé est choisi en fonction de l'hôte dans lequel il sera transféré; il peut s'agir de tout vecteur comme un plasmide. Ces acides nucléiques et vecteurs sont utiles pour produire les peptides et les composés de formule (IV) ou partie de ceux-ci de nature protéique dans un hôte cellulaire. La préparation de ces vecteurs ainsi que la production ou l'expression dans un hôte des peptides ou des composés de formule (IV) peuvent être réalisées par les techniques de biologie moléculaire et de génie génétique bien connues de l'homme du métier.

Les compositions contenant des composés de formule (IV) et avantageusement un véhicule pharmaceutiquement acceptable peuvent être administrées par différentes voies comme par exemple de manière non limitative, les voies intraveineuse, intramusculaire, sous cutanée, etc... .

Les peptides de formule (I), (II) ou (III) permettent de faire traverser la barrière hématoencéphalique à une substance active qui ne passe pas ou peu cette barrière. Ils peuvent donc être utilisés, comme proposé précédemment dans le traitement, la prévention ou le diagnostic d'une maladie affectant le SNC, mais aussi dans le cadre d'études menées sur des drogues diverses avec des modèles de barrière hématoencéphalique.

A titre de substance active, l'invention envisage notamment des protéines, comme des polypeptides ou peptides, des anticorps ou partie d'anticorps, des acides nucléiques et oligonucléotides ou des ribozymes, ou encore, bien entendu des molécules chimiques actives pour le traitement ou la prévention de pathologies humaines ou animales du SNC, comme par exemple et de manière non limitative des antitumoraux, des antiviraux, des antidépresseurs, des analgésiques, etc... .

Dans le domaine du diagnostic, la substance active peut être un marqueur radioactif, un marqueur coloré, ou tout autre moyen ou substance capable de révéler un métabolisme ou une pathologie du SNC.

Les affections du SNC, dont le diagnostic, le traitement ou la prévention sont envisagés dans le cadre de la présente invention sont par exemple de manière non limitative, les cancers du cerveau, la maladie d'Alzeihmer, la maladie de Parkinson, la dépression, la douleur, les méningites, etc... .

L'invention concerne donc tout particulièrement l'utilisation des composés de formule (IV) pour la préparation d'un médicament destiné au traitement ou à la prévention d'une affection choisie parmi : les cancers du cerveau, la maladie d'Alzeihmer, la maladie de Parkinson, la dépression, la douleur, les méningites.

D'autres avantages et caractéristiques de l'invention apparaîtront dans les exemples qui suivent concernant la préparation de composés de formule (IV) où la substance active est la doxorubicine, la dalargine, la penicilline et leur pénétration dans le cerveau conformément à l'utilisation de peptides linéaires selon l'invention.

### Exemple I : Pénétration de la doxorubicine.

### I - Conditions expérimentales.

### 1) Synthèse Chimique.

Plusieurs peptides ont été synthétisés et leur internalisation a été testée dans plusieurs lignées cellulaires. De façon générale, les propriétés physico-chimiques des peptides ont été modifiées, et les résultats obtenus montrent que suivant la modification, certains peptides pénètrent beaucoup mieux que d'autres, comme les peptides des composés No. 1 et 2 du tableau I ci-après. Il a également été observé que certains peptides pénètrent plus rapidement dans un type cellulaire que dans d'autres, ce qui indique un tropisme cellulaire.

### a) Préparation de Doxorubicine-Succ-Peptides.

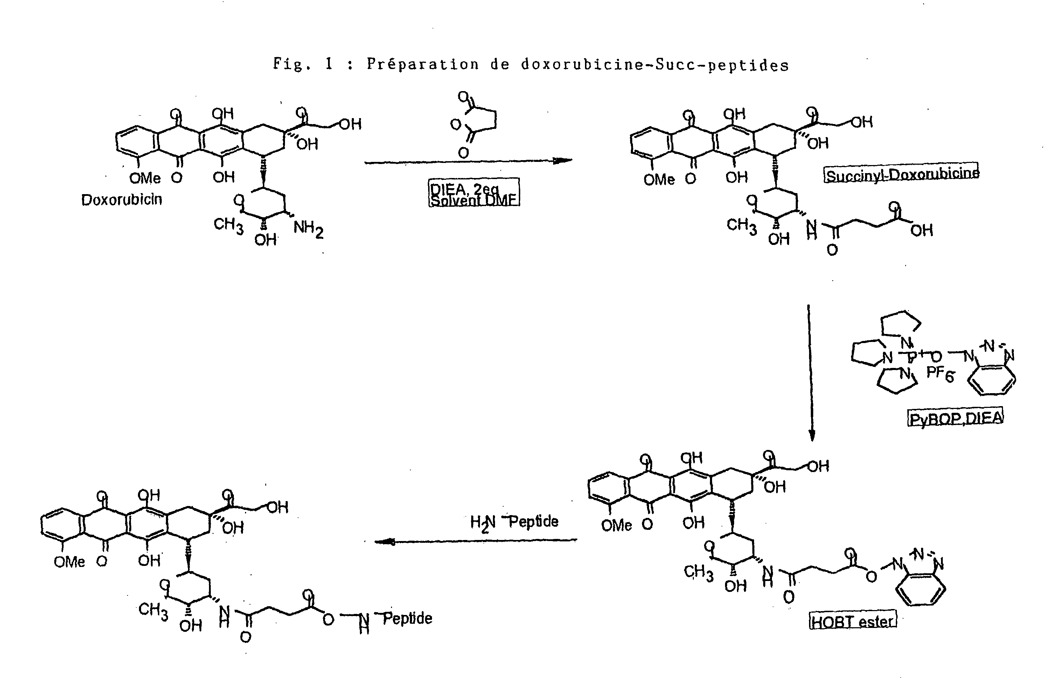

Le couplage de la doxorubicine sur un peptide par l'intermédiaire du maillon succinique est effectué en 3 étapes comme montré sur la figure 1 en annexe.

Au chlorhydrate de doxorubicine (1 eq), solubilisé dans diméthylformaminde (DMF) en présence de Disopropyléthylamine (DIEA, 2 eq) est ajouté l'anhydride succinique (1,1eq, dissous dans DMF).

Après une incubation de 20 min à température ambiante, l'hémisuccinate de doxorubicine ainsi formé est ensuite activé par addition de PyBOP (Benzotriazol-1-yl-oxopyrrolidinephosphonium Hexafluorophosphate 1,1eq dans DMF) et DIEA (2 eq). Ce second mélange réactionnel est incubé 20 min.

Le peptide (1,2 eq dans DMF) est ensuite ajouté au mélange réactionnel, et se couple spontanément sur l'hémisuccinate de doxorubicine activé au cours d'une incubation supplémentaire de 20 min.

Le produit de couplage est ensuite purifié sur HPLC (Chromatographie liquide haute pression) préparative, puis lyophilisé.

Chacune des étapes, ainsi que le produit final sont contrôlés par HPLC analytique et spectrométrie de masse.

### b) Préparation de Doxorubicine-SMP-3MP-Peptide.

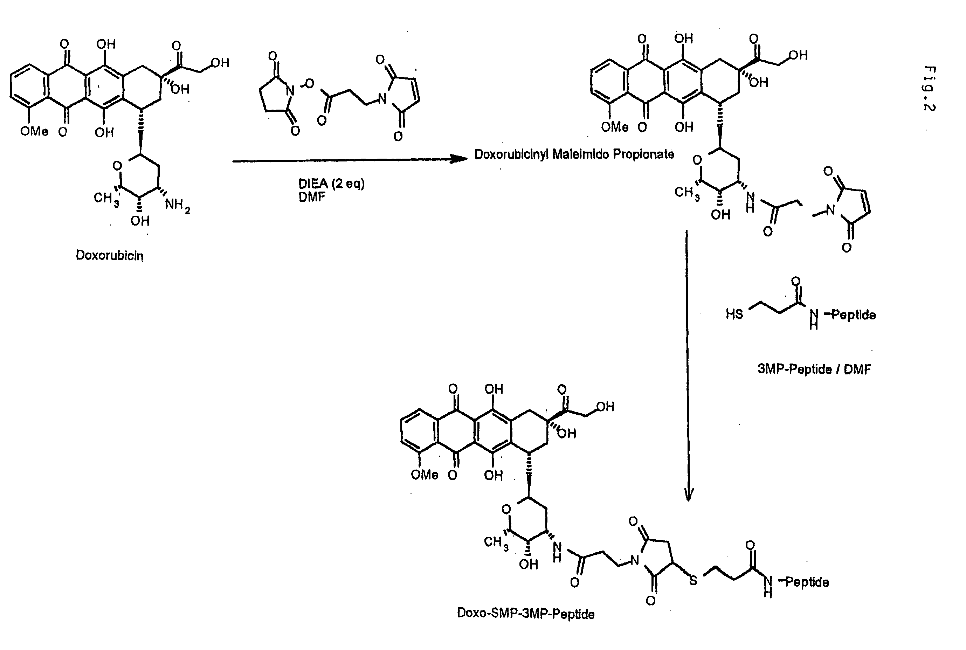

Le couplage de la doxorubicine sur un peptide porteur de fonction thiol est effectué en 2 étapes comme montré sur la figure 2 en annexe.

Au chlorhydrate de doxorubicine (1 eq), solubilisé dans diméthylformaminde (DMF) en présence de Disopropyléthylamine (DIEA, 2 eq) est ajouté le N-hydroxy-Succinimidyl-Maleimido-Propionate (SMP, 1 eq dans dissous dans DMF).

Le peptide porteur d'une fonction thiol (1,2 eq dans DMF) est ensuite ajouté au mélange réactionnel, et se couple spontanément sur le maléimidopropionate de doxorubicine au cours d'une incubation supplémentaire de 20 minutes.

Le produit de couplage est ensuite purifié sur HPLC préparative, puis lyophilisé.

Chacune des étapes, ainsi que le produit final sont contrôlés par HPLC analytique et spectrométrie de masse.

### 2) Produits testés.

Les produits testés sont rapportés dans le tableau I ci-dessous.

**Tableau I**

| Composé | |
|---|---|
| No. 1 (Doxo-SynB1) | doxo-CO-(CH2)₂-CO-RGGRLSYSRRRFSTSTGR |
| No. 2 | doxo-SMP-3MP-rqikiwfgnrrmkwkk |
| doxo : doxorubicine | |
| CO-(CH2)₂-CO : Linker succinate | |
| SMP-3MP : Linker Succinimydyl Maleimido-Propionate-3-MercaptoPropionate | |
| Lettres minuscules : Acides aminés sous forme d. | |

### 3) Perfusion Cérébrale in situ.

### a) Perfusion.

Il s'agit d'une méthode rapide et sensible pour évaluer la pénétration de divers composés dans le système nerveux central (Takasato et al., 1984, Am. J. Physiol. 247, 484-493 ; Allen et al., 1997, Pharm Res. 14, 337-341). Des rats mâles Sprague-dawley de 2 mois (250-350g, Iffa-Credo ; l'Arbresle, France) sont anesthésiés. Après exposition de la carotide commune, l'artère carotide externe droite est liée au niveau de la bifurcation avec la carotide interne et la carotide commune est liée entre le coeur et le site d'implantation du cathéter (cathéter polyéthylène, ID :0.76). Celui-ci , préalablement rempli par une solution d'héparine (100 unités/ml) est inséré dans la carotide commune. Les rats sont perfusés avec le tampon de perfusion (128 mM NaCl, 24 mM NaHCO₃, 4,2 mM KCl, 2,4 mM NaH₂PO₄, 1, 5 mM CaCl₂, 0,9 mM MgSO₄, et 9 mM D-glucose). Ce tampon est filtré puis bullé par un mélange contenant 95% O₂ / 5% CO₂ afin de maintenir le pH proche de 7,4 et d'alimenter le cerveau en oxygène au cours de la perfusion.

Les rats sont perfusés avec le tampon contenant la doxorubicine libre ou les composés No. 1 ou 2. Dans chaque produit la doxorubicine est radiomarquée au carbone 14 (activité spécifique : 9,4 microCi/mg, Amersham, France). Les produits sont perfusés à la concentration de 0,33 microCi/ml ou 0,035 mg/rat.

Juste avant le début de la perfusion, le coeur est arrêté par section des ventricules, ceci afin d'éviter au cours de la perfusion un reflux du perfusat. L'hémisphère droit est alors perfusé à une vitesse de 10 ml/min pendant 60 secondes après quoi le rat est décapité.

### b) Rincage.

Pour les rats subissant l'étape de rinçage, le cathéter préalablement introduit, comme précédemment, dans la carotide commune est connecté à une vanne à 4 voies opposées (Hamilton, USA) reliée à 2 seringues : l'une contenant le traceur radiomarqué (seringue A) et l'autre le tampon seul (seringue B). Une fois le cathéter en place et les connections correctement réalisées, la cage thoracique du rat est ouverte et le coeur sectionné. Le contenu de la seringue A est alors immédiatement perfusé au débit de 10 ml/min. Au bout de 60 secondes, le contenu de la seringue B est à son tour injecté au même débit. Après 30 secondes de rinçage, le rat est décapité.

### c) Dissection du cerveau.

Après décapitation, le cerveau est rapidement prélevé. Ce dernier est disséqué sur la glace en 8 régions : Hypothalamus (HY), Cortex frontal (CF), Mésencéphale (MS), Cortex Occipital (CO), Cortex Pariétal (CP), Thalamus (TH), Hippocampe (HP), Striatum (ST), qui sont déposées dans des fioles en verre préalablement tarées, puis pesées. Ces structures ainsi que 50 microlitre du perfusat sont digérées pendant 2 heures dans 1 ml de soluène à 60°C. Un cocktail scintillant (10 ml ; Pico-fluor ; Packard) est ajouté à chacun des échantillons et la quantité de traceurs qu'ils contiennent est mesurée par un double comptage en scintillation liquide (Packard, Tricarb, 1900TR).

### d) Déplétion capillaire.

Cette méthode permet de mesurer la répartition des produits entre le parenchyme cérébral et les cellules endothéliales Triguero et al., 1990, J. Neurochem. 54, 1882-1888). Après 60 secondes de perfusion suivies ou non de 30 secondes de rinçage, l'hémisphère droit est prélevé, débarrassé de ses méninges et plexus choroïdes, puis homogénéisé dans 3,5 ml de tampon Hepes (en mM : 10 Hepes, 141 NaCl, 4 KCl, 1 NaH₂PO₄, 2,8 CaCl₂ , 1 MgSO₄ et 10 mM D-glucose, pH = 7.4). Après broyage au potter, 4 ml de solution contenant 4% de Dextran (PM : 76900) sont ajoutés et le mélange vigoureusement agité afin d'obtenir une concentration finale de 20%. Toutes ces opérations sont réalisées à 4°C en moins de 5 minutes. Après avoir prélevé un échantillon de l'homogénat ainsi obtenu, ce dernier est alors mis à centrifuger pendant 15 min à 5400 g afin de séparer les cellules endothéliales présentes dans le culot du parenchyme cérébral resté dans le surnageant. Les résultats sont exprimés par les volumes de distribution dans le surnageant et le culot.

### 4) Injections intraveineuses.

Les souris NMRI-nude sont injectées par voie intraveineuse avec le composé No. 1 ou la doxorubicine seule à une dose de 2,5 mg/Kg (équivalent en doxorubicine). La doxorubicine est marquée au carbon 14 (environ 0,5 microCi sont injectés par souris). Après 1, 5, 15, 30, 60, 180, 480, et 1360 minutes, les souris sont sacrifiées. Les organes sont ensuite prélevés et comptés. La quantité de radioactivité dans chaque organe est ensuite exprimée en quantité de produit par gramme d'organe. Dans cette étude, nous avons utilisé cinq souris par temps.

Dans le cas du composé No. 2, la souris CD1 est injectée par voie intraveineuse avec le composé No. 2 ou la doxorubicine libre à une dose de 2 mg/Kg (équivalent en doxorubicine). La doxorubicine est marquée au carbon 14 (environ 3 microCi sont injectés par souris). Après des temps 15 minutes, 2 heures et 8 heures, la souris est sacrifiée et la quantité de produit dans chaque organe est analysée par la technique " Whole-body autoradiography ". La quantité de radioactivité dans chaque organe est ensuite exprimée en quantité de produit par gramme d'organe. Dans cette expérience, nous avons utilisé une souris par groupe.

### II - RÉSULTATS.

### 1) Perfusion Cérébrale in situ.

### a) Tolérance des produits.

Dans un premier temps, l'effet des produits testés sur l'intégrité de la BHE a été observé, grâce au volume de distribution du [³H]-sucrose, qui est une petite molécule ne pénétrant pas dans le système nerveux central pour des temps d'exposition courts. On estime que ce volume ne doit pas excéder 18 µg/ml. Au delà, on conclut à une perméabilité anormale de la BHE.

La doxorubicine, les composés No. 1 et 2 ont été injectés en présence du sucrose et l'intégrité de Ia BHE est mesurée. Le tableau II ci-dessous rapporte l'effet de la perfusion des produits-sur l'intégrité de la BHE.

**Tableau II**

| Composé | Dose perfusée | Etat de la BHE |
|---|---|---|
| doxorubicine | 0,07 mg | Intègre |
| No. 1 (Doxo-SynB1) | 0,05 mg 0,8 mg | Intègre Intègre |
| No. 2 | 0,05 mg 0,2 mg 0,8 mg | Intègre Intègre Anormal |

Le composé No. 1 ne provoque pas d'ouverture anormale de la BHE même à des doses de 0,8 mg. Par contre le composé No. 2, il y a une ouverture de la BHE à des doses supérieures à 0,2 mg. Par conséquent, les travaux ont été réalisés avec des produits à des doses de 0,05 mg.

### b) Pénétration des Produits.

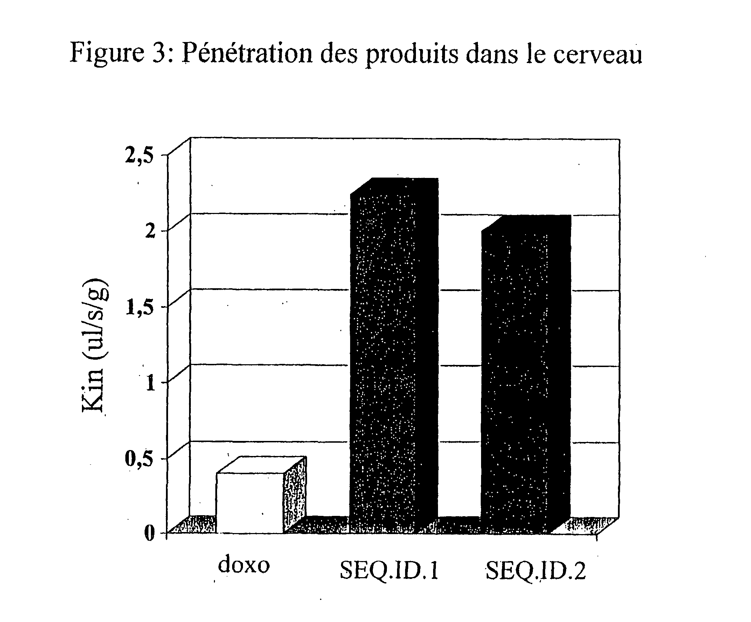

Cette étude a consisté à comparer la pénétration dans la BHE de la doxorubicine seule avec la doxorubicine vectorisée dans les composés No. 1 et 2. Après 60 secondes de perfusion dans le tampon, la pénétration des produits est estimée par la constante d'influx ou Kin en microl/sec/g. La figure 3 représente la pénétration des produits dans le cerveau. On observe que la vectorisation de la doxorubicine par les deux vecteurs augmente son passage dans le cerveau de 5 à 7 fois après une perfusion de 60 secondes dans du tampon.

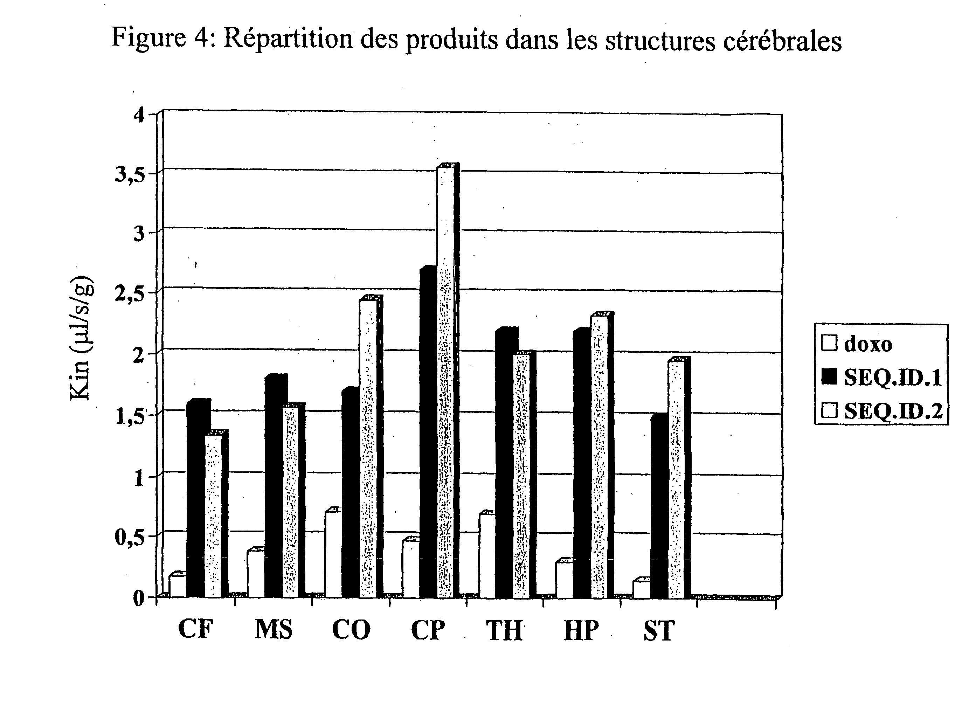

Dans une autre expérience, le cerveau a été disséqué en 8 régions comme décrit précédemment et la quantité de produit dans chaque région a été mesurée. La figure 4 en annexe rapporte la pénétration de ces produits dans le cerveau. On observe que. la pénétration des composés No. 1 et 2 est 5 à 7 fois supérieure à celle de la doxorubicine libre et ce, quelle que soit les structures cérébrales considérées.

### c) Pénétration des produits après rincage.

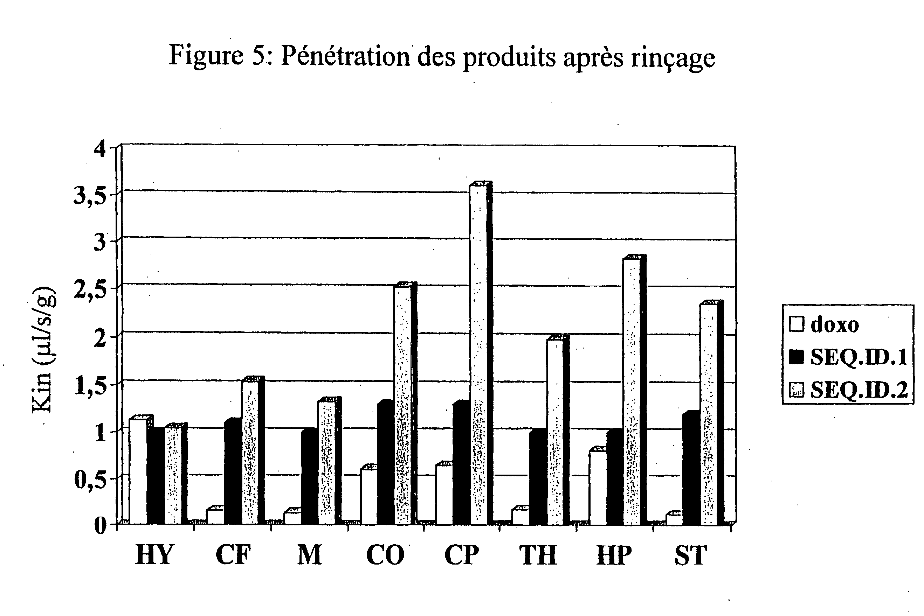

Le rinçage des capillaires cérébraux par perfusion de tampon sans traceur pendant 30 secondes permet d'éliminer la fraction du produit étudié éventuellement adhérente à la membrane luminale des cellules endothéliales. La figure 5 en annexe rapporte les résultats de la pénétration des produits après rinçage. On abserve pour la doxorubicine libre une diminution de la constante d'influx de 25% environ. Pour la doxorubicine vectorisée, cette diminution est de 45% pour le composé No.1 et de 10% pour le composé No. 2. Finalement, la pénétration des composés No. 1 et 2 est respectivement augmentée de 4 et 7 fois par rapport à la doxorubicine libre.

### d) Répartition des produits après déplétion capillaire.

Cette méthode permet de mesurer la répartition des produits entre le parenchyme cérébral et les cellules endothéliales. La déplétion capillaire est effectuée après perfusion de 60 secondes suivie d'un rinçage de 30 secondes. Les volumes de distribution (Vd) dans les cellules endothéliales et le parenchyme cérébral sont exprimés en microlitre/g.

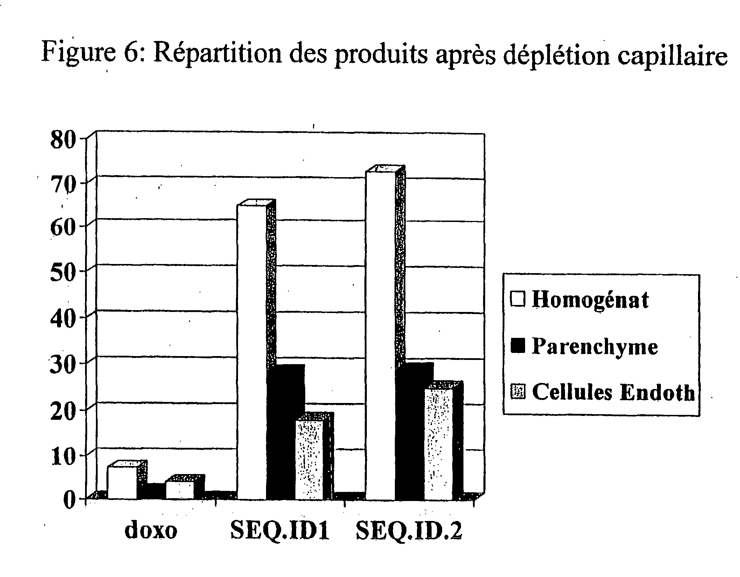

La figure 6 en annexe indique la répartition des produits après déplétion capillaire. On observe pour la doxorubicine libre un Vd dans le parenchyme cérébral de 1,75 µl/g, pour le composé No. 1 un Vd de 28,5 µl/g et pour le composé No.2 un Vd de 29,5 µl/g. Ces résultats montrent que la pénétration de la doxorubicine vectorisée (composés No. 1 et 2) dans le parenchyme cérébral est considérablement augmentée par rapport à celle de la doxorubicine libre. On retrouve environ 60% des composés No. 1 et 2 dans le parenchyme cérébral 1 minute après la perfusion cérébrale des molécules suivie de 30 secondes de rinçage des capillaires cérébraux.

### 2) Injection intraveineuse.

L'étude du passage d'une substance au travers de la BHE nécessite l'emploi de plusieurs approches complémentaires. La perfusion cérébrale permet des mesures sur des temps très courts. L'injection intraveineuse permet une évaluation globale des pharmacocinétiques chez l'animal sur des temps longs. La molécule radioactive est introduite dans la circulation sanguine et se distribue dans l'organisme. Une certaine quantité de cette molécule pénètre dans le cerveau où elle est mesurée à des temps déterminés.

### a) Avec le composé No. 1.

Après injection en intraveineuse du composé No. 1, les souris nude sont sacrifiées à différents temps et la radioactivité totale dans le cerveau est comptée et exprimée en quantité de produit par gramme de cerveau. Le tableau III ci-dessous indique la quantité de doxorubicine et de composé No. 1 dans le cerveau.

**Tableau III**

| Groupe | Produit | Dose (mg baseDXR/Kg) | Temps (min) | Quantité de produit (microg/g cerveau) |
|---|---|---|---|---|
| 1 | doxorubucine | 2,5 | 1 | 0,14 |
| | | | 5 | 0,05 |
| | | | 15 | 0,04 |
| | | | 30 | 0,05 |
| | | | 60 | 0,04 |
| | | | 180 | 0,03 |
| | | | 480 | 0,04 |
| | | | 1360 | 0,01 |
| 2 | Composé No.1 | 2,5 | 1 | 0,48 |
| | | | 5 | 0,18 |
| | | | 15 | 0,42 |
| | | | 30 | 0,25 |
| | | | 60 | 0,12 |
| | | | 180 | 0,03 |
| | | | 480 | 0,02 |
| | | | 1360 | 0,01 |

La vectorisation a permis d'améliorer de façon significative le passage de la doxorubicine à travers la barrière hémato-encéphalique. Cette accumulation est observée non seulement pour des temps courts mais aussi pour des temps longs allant jusqu'à 3 heure post-administration. Le tableau IV ci-dessous indique le rapport de la doxorubicine vectorisée (composé No. 1) par rapport à la doxorubicine seule.

**Tableau IV**

| Temps (min) | Rapport Composé No. 1 / doxorubicine |
|---|---|
| 1 | 3,4 |
| 5 | 3,6 |
| 15 | 10,5 |
| 30 | 5 |
| 60 | 3 |
| 180 | 1 |

### b) Avec le composé No. 2.

Après injection intraveineuse du composé No. 2, les souris CD-1 sont sacrifiées après des temps de 15 minutes, 2 heures et 8 heures. La radioactivité totale dans le cerveau est analysée par la méthode "Whole body autoradiography" et- exprimée en quantité de produit par gramme de cerveau. La tableau V ci-dessous indique la quantité de doxorubicine et de coposé No. 2 dans le cerveau.

**Tableau V**

| Groupe | Produit | Dose (mg baseDXR/Kg) | Temps (min) | Quantité de produit (ug/g cerveau) |
|---|---|---|---|---|
| 1 | doxorubucine | 2 | 15 | 1,24 |
| | | | 120 | 0,98 |
| | | | 480 | 0,67 |
| 2 | Composé No.2 | 2 | 15 | 9,49 |
| | | | 120 | 4,73 |
| | | | 480 | 4,52 |

La vectorisation a permis d'améliorer de façon significative le passage de la doxorubicine à travers la barrière hémato-encéphalique. Cette accumulation est observée pour des temps longs allant jusqu'à 8 heures post-administration. Le tableau VI ci-dessous représente le rapport de la doxorubicine vectorisée (composé No. 2) par rapport à la doxorubicine seule.

**Tableau VI**

| Temps (min) | Rapport Composé No. 2 / doxorubicine |
|---|---|
| 15 | 7,65 |
| 120 | 4,83 |
| 480 | 6,75 |

### Exemple II : Pénétration de la dalargine.

### 1) Produits testés.

Les produits testés dans cet exemple sont rapportés dans le tableau VII ci-dessous.

**Tableau VII**

| composé | |
|---|---|
| 3 : dalarqine | Y-(D)A-GFLR |
| 4 : dal-SynB1 | Y-(D)A-GFLR-S-S-RGGRLSYSRRRFSTSTGR |
| dal : dalargine | |
| S-S : Linker dissulfide | |
| (D) : acide aminé en forme d. | |

### 2) Pénétration des Produits.

Cette étude a consisté à comparer la pénétration dans la BHE de la dalargine seule avec la dalargine vectorisée. La dalargine est un peptide analgésique. La dalargine a été liée par un pont dissulfure au peptide vecteur du tableau VII. Cette forme de liaison hydrolysable a été retenu car il a été démontré dans la littérature que ces ponts dissulfures sont stables dans le plasma mais dès que le produit passe la BHE, le pont est hydrolysé libérant ainsi la drogue.

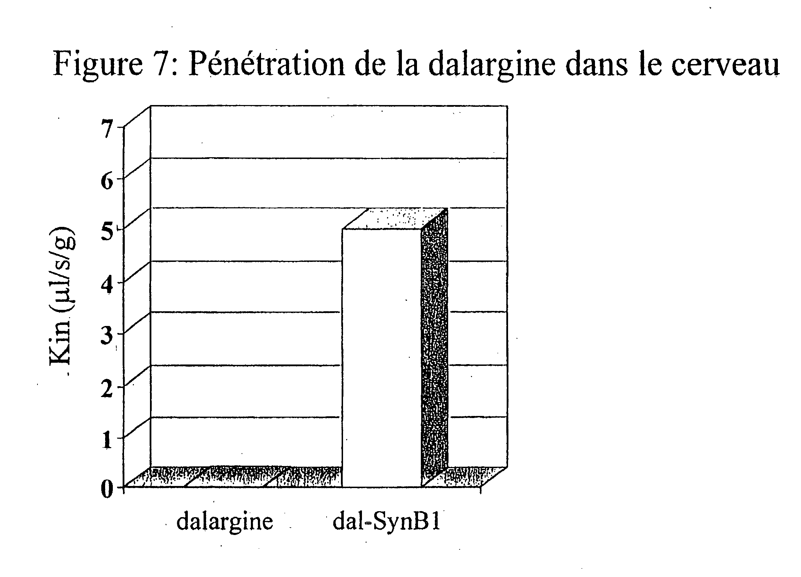

Après 60 secondes de perfusion dans le tampon, la pénétration des produits est estimée par la constante d'influx ou Kin en µl/sec/g. La figure 7 montre que la vectorisation de la dalargine par le peptide vecteur SynB1 augmente considérablement son passage dans le cerveau après une perfusion de 60 secondes dans du tampon.

### 3) Activité Biologique.

Dans le cadre de cette étude, l'activité biologique de la dalargine seule a été comparée à l'activité biologique de la dalargine vectorisée avec SynB1. A cet effet, le modèle « Hot Plate » chez la souris a été utilisé. Dans ce modèle, la souris est placée sur une plaque chauffante et le temps que la souris met pour réagir à la chaleur est mesuré « temps de latence ».

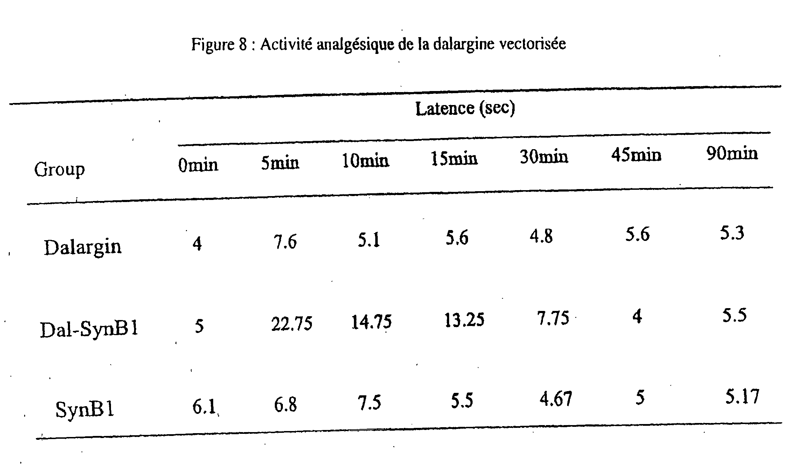

Il a été injecté aux souris par voie intraveineuse 2 mg/Kg de. chaque produit (la dose correspondant à la quantité de dalargine). Après des temps variant de 0 à 90 minutes, le temps de latence a été mesuré. La figure 8 montre qu'après l'injection de la dalargine seule, aucun effet n'est obtenu. Le temps de latence est constant. Par contre quand la dalargine vectorisée est injectée, on observe une augmentation de temps de latence surtout pour des temps allant de 5 à 30 minutes après administration du produit. Par exemple, 5 minutes après injection, le temps de latence de la dalargine vectorisée est de 22,75 sec alors que pour la dalargine seule, il n'est que de 7,6 sec. Ceci indique clairement qu'il y a augmentation de l'activité analgésique de la dalargine vectorisée.

Il a également été vérifié que cet effet n'est pas dû au peptide tout seul. Pour cela, le vecteur SynB1 seul a été injecté et le temps de latence a été mesuré. Les résultats sont comparables à ceux de la dalargine seule, indiquant que le vecteur seul n'a aucune activité analgésique.

### Exemple III : Pénétration de la doxorubicine.

Cet exemple concerne un autre peptide vecteur que celui de l'exemple 1.

### 1) Produits testés.

Les produits testé dans cet exemple sont rapportés dans le tableau VIII ci-dessous.

**Tableau VIII**

| Composé | |
|---|---|
| doxo | doxorubicine |
| N°5 : doxo-SynB 3 | doxo-CO-(CH2)₂-CO- RRLSYSRRRF |
| Doxo : doxorubicine | |
| CO-(CH2)₂-CO : Linker succinate | |

### 2) Pénétration des Produits.

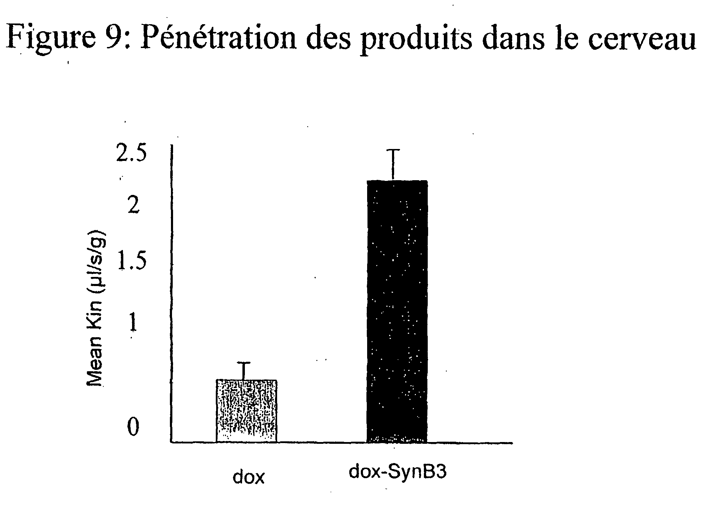

Cette étude a consisté à comparer la pénétration dans la BHE de la doxorubicine seule avec la doxorubicine vectorisée. Après 60 secondes de perfusion dans le tampon, la pénétration des produits est estimée-par la constante d'influx ou Kin en µl/sec/g. La figure 9 montre que la vectorisation de la doxorubicine par le vecteur SynB3 augmente son passage dans le cerveau de 5 fois après une perfusion de 60 secondes dans du tampon.

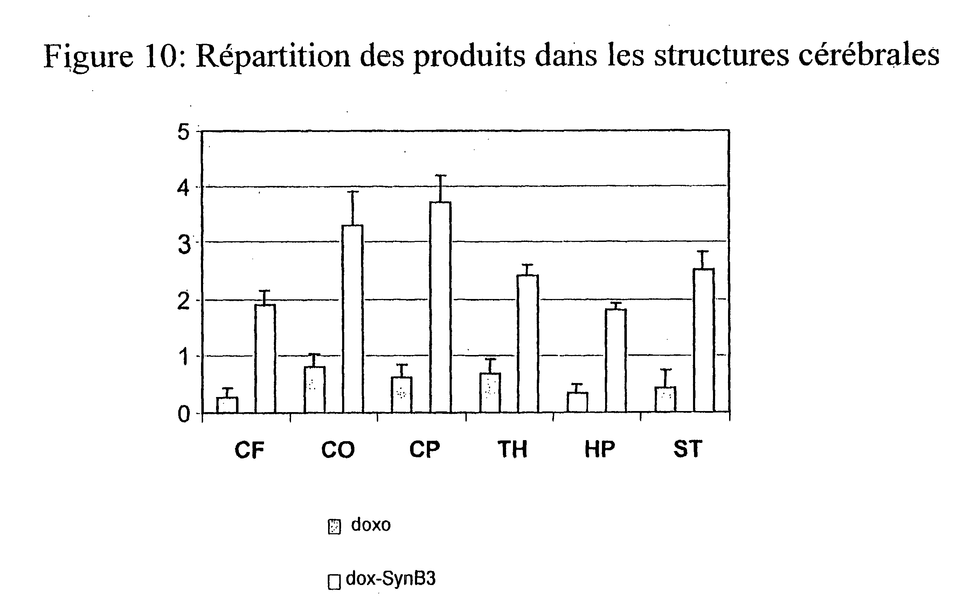

Dans une autre expérience, le cerveau a été disséqué en 6 régions comme décrit ci-dessus et la quantité de produit dans chaque région a été mesurée. La figure 10 montre une pénétration du composé N°2 supérieure à celle de la doxorubicine libre et ce, quelle que soit les structures cérébrales considérées.

### 3) Répartition des produits après déplétion capillaire.

Cette méthode permet de mesurer la répartition des produits entre le parenchyme cérébral et les cellules endothéliales. La déplétion capillaire est effectuée après perfusion de 60 secondes suivie d'un rinçage de 30 secondes. Les volumes de distribution (Vd) dans les cellules endothéliales et le parenchyme cérébral sont exprimés en µl/g.

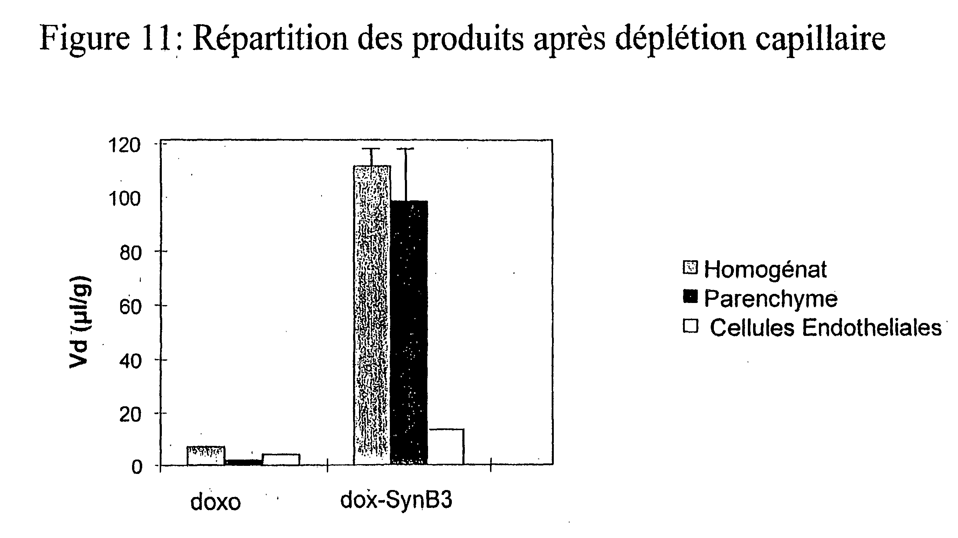

On observe sur la figure 11 pour la doxorubicine libre un Vd dans le parenchyme cérébral de 1,75 µl/g, et un Vd de 98,32 µl/g et pour la doxorubicine vectorisée. Ceci indique que la pénétration de la doxorubicine vectorisée dans le parenchyme cérébral est considérablement augmentée 50 fois par rapport à celle de la doxorubicine libre.

### Exemple IV : Pénétration de la pénicilline.

### 1) Produits testés.

Les produits testés dans cet exemple sont rapportés dans le tableau VII ci-dessous.

**Tableau IX**

| Composé | |
|---|---|
| PNC | benzylpenicilline |
| N°6 : PNC-SynB1 | PNC-linker-RGGRLSYSRRRFSTSTGR |

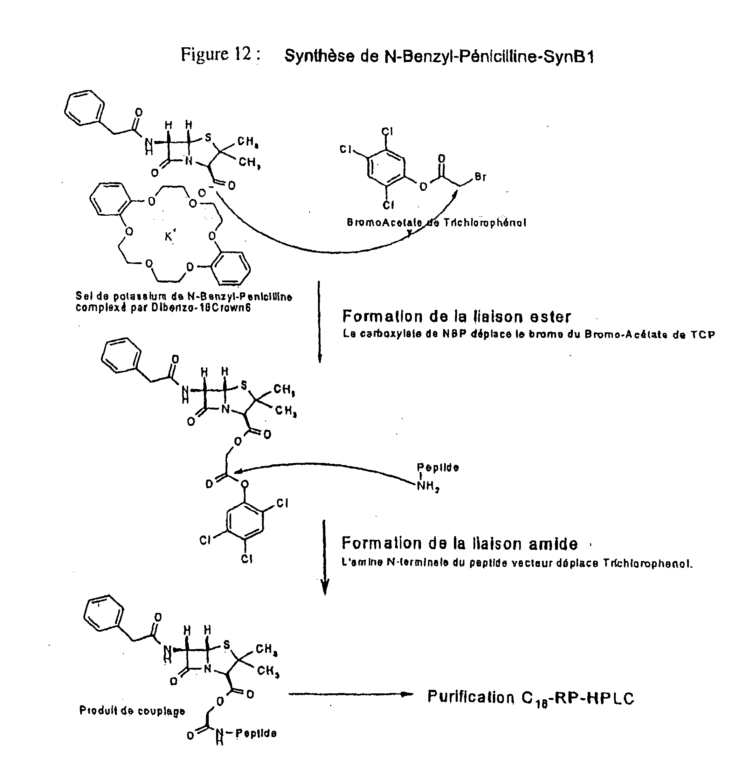

Le schéma du couplage de la benzylpénicilline avec le vecteur SynB1 est représenté dans la figure 12. La N-benzyl pénicilline (NBP) a été couplée au vecteur SynB1 par l'intermédiaire d'un maillon glycolamidique. Dans une première étape, le carboxylate libre de NBP est couplé par liaison ester sur le bromo-acétate de trichlorophénol. Le vecteur est ensuite couplé par liaison amide sur son extrémité N-terminale, avec départ de trichlorophénol. Le produit de couplage est purifié par chromatographie sur phase réverse puis lyophilisé.

### 2) Pénétration des Produits.

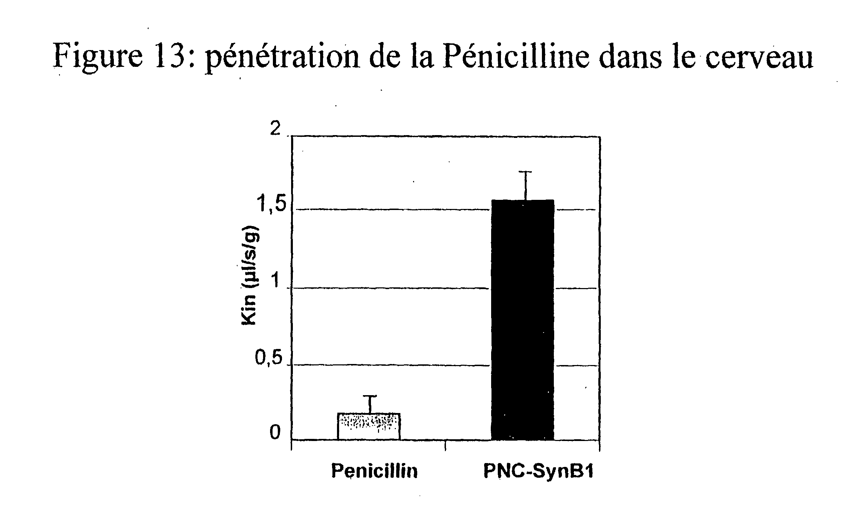

La pénétration dans la BHE de la benzylpénicilline seule a été comparée celle de avec la benzylpénicilline vectorisée (composé N°6). Après 60 secondes de perfusion dans le tampon, la pénétration des produits radiomarqués est estimée par la constante d'influx ou Kin en µl/sec/g. La figure 13 montre que la vectorisation de la pénicilline par le vecteur augmente son passage dans le cerveau d'environ 9 fois après une perfusion de 60 secondes dans du tampon.

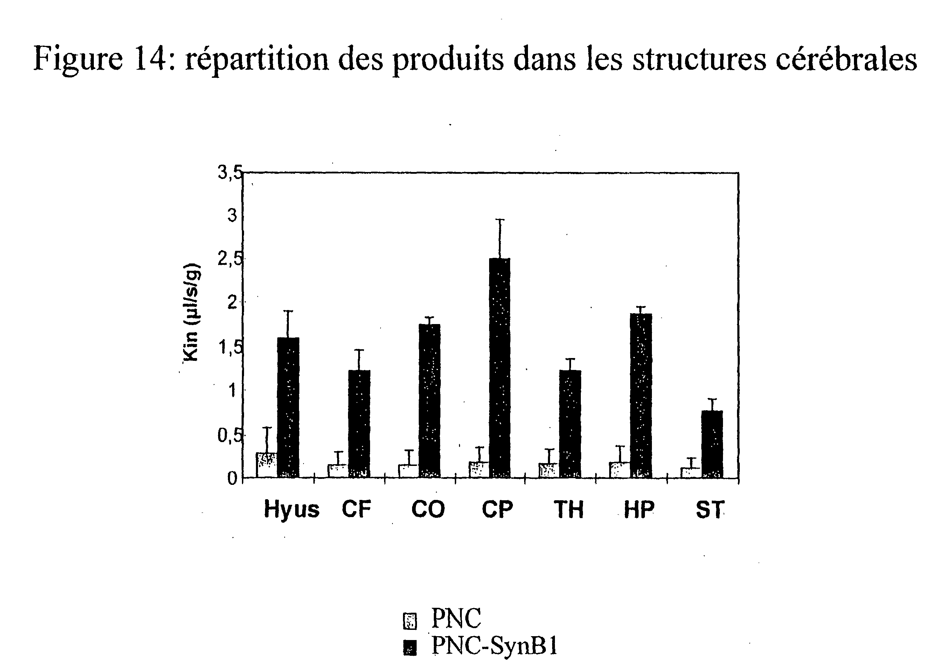

Dans une autre expérience, après 30 secondes de perfusion dans le tampon, le cerveau a été disséqué en plusieurs régions comme décrit précédemment et la quantité de produit dans chaque région a été mesurée. La figure 14 montre qu'on observe une pénétration du composé N°6, six à quatorze fois supérieure à celle de la pénicilline libre et ce, dépendant de la structure cérébrale considérée.

### 3) Répartition des produits après déplétion capillaire.

Cette méthode permet de mesurer la répartition des produits entre le parenchyme cérébral et les cellules endothéliales. La déplétion capillaire est effectuée après perfusion de 30 secondes suivie d'un rinçage de 30 secondes. Les volumes de distribution (Vd) dans les cellules endothéliales et le parenchyme cérébral sont exprimés en µl/g.

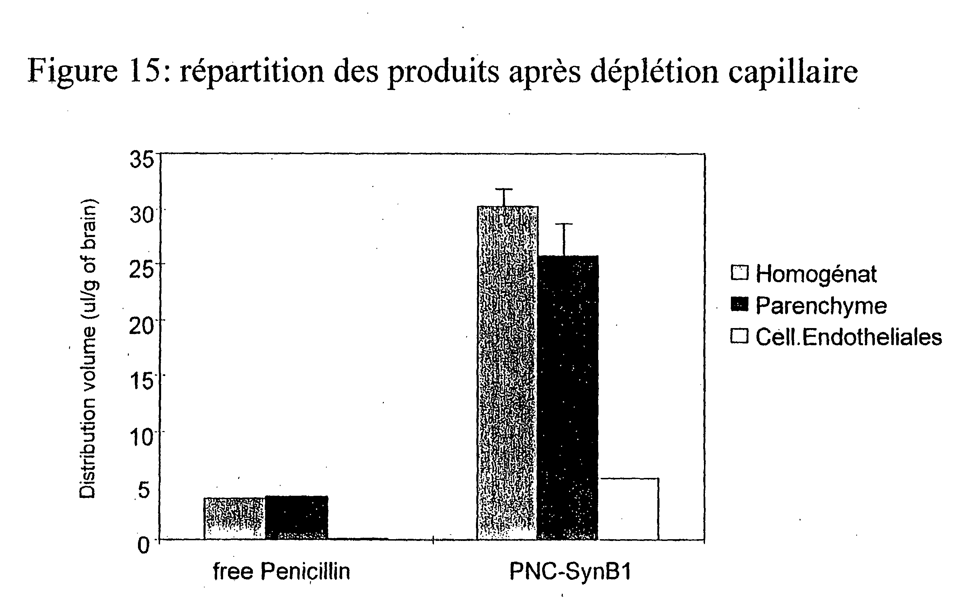

On observe sur la figure 15, pour la benzylpénicilline libre un Vd dans le parenchyme cérébral de 3,94 µl/g et un Vd de 25,76 µl/g pour la benzylpénicilline vectorisée. Ceci indique que la pénétration de la benzylpénicilline vectorisée dans le parenchyme cérébral est considérablement augmentée par rapport à celle de la benzylpénicilline libre.

## Revendications

1. Utilisation d'un peptide linéaire couplé à une substance active en diagnostic ou thérapie d'une affection du SNC pour la préparation d'un médicament capable de traverser la barrière hémotoencéphalique à utiliser en diagnostic ou thérapie d'une affection localisée au niveau du SNC, ledit peptide répondant à l'une des formules (II) ou (III) suivantes :
BXXBXXXXBBBXXXXXXB (II)
BXXXBXXXBXXXXBBXB (III),
dans lesquelles formules:
- les groupes B, identiques ou différents, représentent un résidu d'acide aminé dont la chaîne latérale porte un groupement basique, et
- les groupes X, identiques ou différents, représentent un résidu d'acide aminé aliphatique ou aromatique,
ou lesdits peptides de formules (II) et (III) sous forme rétro, constitués d'acides aminés de configuration D et/ou L, ou un fragment de ceux-ci constitué d'une séquence d'au moins 5 et de préférence d'au moins 7 acides aminés successifs des peptides de formules ou (III).

2. Utilisation d'un peptide linéaire couplé à une substance active en diagnostic ou thérapie d'une affection du SNC pour la préparation d'un médicament capable de traverser la barrière hémotoencéphalique à utiliser en diagnostic ou thérapie d'une affection localisée au niveau du SNC, ledit peptide répondant à l'une des formules (II) ou (III) suivantes :
BXXBXXXXBBBXXXXXXB (II)
BXXXBXXXBXXXXBBXB (III),
dans lesquelles formules:
- B est choisi parmi l'arginine, la lysine, l'acide diaminoacétique, l'acide diaminobutyrique, l'acide diaminopropionique, l'ornithine, et
- X est choisi parmi la glycine, l'alanine, la valine, la norleucine,. l'isoleucine, la leucine, la cystéine, la cystéine^{Acm}, la penicillamine, la méthionine, le serine, la thréonine, l'asparagine, la glutamine, la phényalanine, l'histidine, le tryptophane, la tyrosine, la proline, l'Abu, l'acide amino-1-cyclohexane carboxylique, l'Aib, la 2-aminotétraline carboxylique, la 4-bromophényalanine, tert-Leucine, la 4-chlorophénylalanine, la bêta-cyclohexyalanine, la 3,4-dichlorophényalanine, la 4-fluorophényalanine, l'homoleucine, la bêta-homoleucine, l'homophényalanine, la 4-méthylphényalanine, la 1-naphtyalanine, la 2- naphtyalanine, la 4-nitrophényalanine, la 3-nitrotyrosine, la norvaline, la phénylglycine, la 3-pyridyalanine, la [2-thiényl]alanine ;
ou lesdits peptides de formules (II) et (III) sous forme rétro, constitués d'acides aminés de configuration D et/ou L, ou un fragment de ceux-ci constitué d'une séquence d'au moins 5 et de préférence d'au moins 7 acides aminés successifs des peptides de formules (II) ou (III).

3. Utilisation de composés répondant à la formule (IV) suivante :
A (-)ₘ (B)ₙ (IV)
dans laquelle
- A représente un peptide tel que défini dans l'une des revendications 1 ou 2,
- B représente une substance active en diagnostic ou thérapie d'une affection du SNC,
- n est 1 ou plus, et de préférence jusqu'à 10 avantageusement jusqu'à 5,
- (-)ₘ représente la liaison, ou linker, entre A et B, où m est 1 ou plus, et de préférence jusqu'à 10 avantageusement jusqu'à 5,
pour la préparation d'un médicament capable de traverser le barrière hémotoencéphalique à utiliser en diagnostic ou thérapie d'une affection localisée au niveau du SNC.

4. Utilisation selon la revendication 3, **caractérisée en ce que** dans la formule (IV), la liaison (-)ₘ entre A et B est une liaison covalente, hydrophobe ou ionique, clivable ou non-clivable dans les milieux physiologiques ou à l'intérieur de la cellule, ou un mélange de celles-ci.

5. Utilisation selon l'une quelconque des revendications 3 ou 4, pour la préparation d'un médicament destiné au traitement ou à la prévention d'une affection choisie parmi : les cancers du cerveau, la maladie d'Alzeihmer, la maladie de Parkinson, la dépression, la douleur, les méningites.

6. Utilisation d'un peptide linéaire couplé à une substance active en diagnostic ou thérapie d'une affection du SNC pour la préparation d'un médicament capable de traverser la barrière hémotoencéphalique à utiliser en diagnostic ou thérapie d'une affection localisée au niveau du SNC, ledit peptide répondant à la formule suivante :
RGGRLSYSRRRFSTSTGR (Syn B1).

7. Utilisation d'un peptide linéaire couplé à une substance active en diagnostic ou thérapie d'une affection du SNC pour la préparation d'un médicament capable de traverser la barrière hémotoencéphalique à utiliser en diagnostic ou thérapie d'une affection localisée au niveau du SNC, ledit peptide répondant à la formule suivante :
RRLSYSRRRF (Syn B3).

## Patentansprüche

1. Einsatz eines linearen Peptids, das an eine aktive Substanz verbunden ist, in einer Diagnose oder Therapie von einer Affektion des ZNS für die Zubereitung eines Medikaments, das fähig ist, die hemotoenzephalische Barriere zu durchqueren, das in einer Diagnose oder Therapie von einer im Bereich des ZNS lokalisierten Affektion eingesetzt wird, wobei das besagte Peptid einer der nachstehenden Formeln (II) oder (III) entspricht:
BXXBXXXXBBBXXXXXXB (II),
BXXXBXXXBXXXXBBXB (III),
wobei in den besagten Formeln:
- die Gruppen B, identisch oder verschieden sind, ein Rest an Aminosäure darstellen, deren Seitenkette eine basische Gruppe trägt,
- und wobei die Gruppen X, identisch oder verschieden sind, einen Rest an alphatischer oder aromatischer Aminosäure darstellen,
oder die besagten Peptide der Formeln (II) und (III) in Retro-Form, bestehend aus Aminosäuren der Konfiguration D und/oder L, oder ein Fragment von diesen vorgenannten bestehend aus einer Sequenz von mindestens 5 und vorzugsweise mindestens 7 aufeinander folgenden Aminosäuren der Peptide der Formeln (II) oder (III).

2. Einsatz eines linearen Peptids, das an eine aktive Substanz verbunden ist, in einer Diagnose oder Therapie von einer Affektion des ZNS für die Zubereitung eines Medikaments, das fähig ist, die hemotoenzephalische Barriere zu durchqueren, das in einer Diagnose oder Therapie von einer im Bereich des ZNS lokalisierten Affektion eingesetzt wird, wobei das besagte Peptid einer der nachstehenden Formeln (II) oder (III) entspricht:
BXXBXXXXBBBXXXXXXB (II),
BXXXBXXXBXXXXBBXB (III),
wobei in den besagten Formeln:
- B zwischen dem Arginin, dem Lysin, der Diaminoessigsäure, der Diaminobuttersäure, der Diaminoproprionsäure, dem Ornithin ausgewählt wird, und
- X zwischen dem Glycin, dem Alalin, dem Valin, dem Norleucin, dem Isoleucin, dem Leucin, dem Cystein, dem Cystein^{Acm}, dem Penicillamin, dem Methionin, dem Serin, dem Threonin, dem Asparagin, dem Glutamin, dem Phenyalanin, dem Histidin, dem Tryptophan, dem Tyrosin, dem Prolin, der Abu, der Amino-1-Cyclohexan-Carbonsäure, der Aib, dem 2-Aminotetralin-Carbon, der 4-Bromophenyalanin, dem tert-Leucin, dem 4-Chlorophenylalanin, dem Betacyclohexyalanin, dem 3,4-Dichlorophenyalanin, dem 4-Fluorophenyalanin, dem Homoleucin, dem Beta-Homoleucin, dem Homophenyalanin, dem 4-Methylphenyalanin, dem 1-Naphtyalanin, dem 2-Naphtyalanin, dem 4-Nitrophenyalanin, dem 3-Nitrotyrosin, dem Norvalin, dem Phenylglycin, dem 3-Pyridyalanin, dem [2-Thienyl]alanin ausgewählt wird;
oder die besagten Peptide der Formeln (II) und (III) in Retro-Form, bestehend aus Aminosäuren der Konfiguration D und/oder L, oder ein Fragment von diesen vorgenannten bestehend aus einer Sequenz von mindestens 5 und vorzugsweise mindestens 7 aufeinander folgenden Aminosäuren der Peptide der Formeln (II) oder (III).

3. Einsatz von Bindungen, die der nachstehenden Formel (IV) entsprechen:
A (-)ₘ (B)ₙ (IV),
in der
- A ein Peptid nach Anspruch 1 oder 2 darstellt,
- B eine aktive Substanz in der Diagnose oder Therapie einer Affektion des ZNS darstellt,
- n 1 oder mehr ist, und vorzugsweise bis zu 10, vorteilhaft bis zu 5,
- (-)ₘ stellt die Bindung oder den Linker zwischen A und B dar, wobei m 1 oder mehr ist, vorzugsweise bis zu 10, vorteilhaft bis zu 5,
für die Zubereitung eines Medikaments, das fähig ist, die hemotoenzephalische Barriere zu durchqueren, das in einer Diagnose oder Therapie von einer im Bereich des ZNS lokalisierten Affektion eingesetzt wird.

4. Einsatz nach Anspruch 3, **dadurch gekennzeichnet, dass** in der Formel (IV) die Bindung (-)ₘ zwischen A und B eine kovalente, hydrophobe oder ionische, spaltbare oder nicht-spaltbare Bindung in den physiologischen Milieus oder im Inneren der Zelle, oder ein Mischung davon ist.

5. Einsatz nach einem der Ansprüche 3 oder 4 für die Zubereitung eines Medikamentes, das vorgesehen ist für die Behandlung oder die Vorbeugung einer Affektion unter: den Krebsleiden am Gehirn, der Alzheimer - Krankheit, der Parkinson - Krankheit, der Depression, den Schmerzen und den Meningitis.

6. Einsatz eines linearen Peptids, das an eine aktive Substanz verbunden ist, in einer Diagnose oder Therapie von einer Affektion des ZNS für die Zubereitung eines Medikaments, das fähig ist, die hemotoenzephalische Barriere zu durchqueren, das in einer Diagnose oder Therapie von einer im Bereich des ZNS lokalisierten Affektion eingesetzt wird, wobei das besagte Peptid der nachstehenden Formel entspricht:
RGGRLSYSRRRFSTSTGR (Syn B1).

7. Einsatz eines linearen Peptids, das an eine aktive Substanz verbunden ist, in einer Diagnose oder Therapie von einer Affektion des ZNS für die Zubereitung eines Medikaments, das fähig ist, die hemotoenzephalische Barriere zu durchqueren, das in einer Diagnose oder Therapie von einer im Bereich des ZNS lokalisierten Affektion eingesetzt wird, wobei das besagte Peptid der nachstehenden Formel entspricht:
RRLSYSRRRF (Syn B3).

## Claims

1. The use of a linear peptide coupled to an active substance for diagnosis or therapy of a disorder affecting the CNS for the preparation of a medicine capable of passing through the hemato-encephalic barrier to be used for diagnosis or therapy of a disorder localized in the CNS, the said peptide satisfying one of the following formulas (II) or (III):
BXXBXXXXBBBXXXXXXB (II)
BXXXBXXXBXXXXBBXB (III),
wherein:
- groups B may be identical or different, and represent an amino acid residue for which the side chain carries a basic group, and
- groups X may be identical or different, and represent a residue of aliphatic or aromatic amino acid, or
the said peptides with formulas (II) and (III) in retro form, constituted of amino acids with a D and/or L configuration, or a moiety of these acids constituted of a sequence of at least 5 and preferably at least 7 successive amino acids of peptides with formulas (II) or (III).

2. The use of a linear peptide coupled to an active substance for diagnosis or therapy of a disorder affecting the CNS for the preparation of a medicine capable of passing through the hemato-encephalic barrier to be used for diagnosis or therapy of a disorder localized in the CNS, the said peptide satisfying one of the following formulas (II) or (III):
BXXBXXXXBBBXXXXXXB (II)
BXXXBXXXBXXXXBBXB (III),
wherein:
- B is chosen among arginine, lysine, diaminoacetic acid, diaminobutyric acid, diaminopropionic acid, ornithine and
- X is chosen among glycine, alanine, valine, norleucine, isoleucine, leucine, cystein, cysteine^{Acm}, penicillamine, methionine, serine, threonine, asparagine, glutamine, phenylalanine, histidine, tryptophan, tyrosine, proline, Abu, carboxylic amino-1-cyclohexane acid, Aib, carboxylic 2-aminotetraline, 4-bromophenylalanine, tert-Leucine, 4-chlorophenylalanine, beta-cyclohexylalanine, 3,4-dichlorophenylalanine, 4-fluorophenylalanine, homoleucine, beta-homoleucine, homophenylalanine, 4-methylphenylalanine, 1-naphthylalanine, 2-naphthylalanine, 4-nitrophenylalanine, 3-nitrotyrosine, norvaline, phenylglycine, 3-pyridylalanine and [2-thienyl]alanine, or
the said peptides with formulas (II) and (III) in retro form, constituted of amino acids with a D and/or L configuration, or a moiety of these acids constituted of a sequence of at least 5 and preferably at least 7 successive amino acids of peptides with formulas (II) or (III).

3. The use of compounds according to the formula (IV) below:
A (-)ₘ (B)ₙ (IV)
Wherein:
- A is a peptide as described above in one of claims 1 or 2,
- B is a substance active in diagnosis or therapy for a disorder of the CNS,
- n is 1 or more, and preferably up to 10, and advantageously up to 5,
- (-)ₘ represents the linker between A and B, where m is 1 or more, and preferably up to 10 and advantageously up to 5,
for the preparation of a medicine capable of passing through the hemato-encephalic barrier to be used in diagnosis or therapy for a disorder localized in the CNS.

4. Use according to claim 3, **characterized in that** in formula (IV), the (-)ₘ linker between A and B is a covalent, hydrophobic or ionic linker, cleavable or non-cleavable in physiological media or inside the cells, or a mixture thereof.

5. Use according to any one of claims 3 or 4, for the preparation of a medicine for the treatment or prevention of an affection chosen among: brain cancer, Alzeihmer's disease, Parkinson's disease, depression, meningitis.

6. The use of a linear peptide coupled to an active substance for diagnosis or therapy of a disorder affecting the CNS for the preparation of a medicine capable of passing through the hemato-encephalic barrier to be used for diagnosis or therapy of a disorder localized in the CNS, the said peptide satisfying the following formula:
RGGRLSYSRRRFSTSTGR (Syn B1).

7. The use of a linear peptide coupled to an active substance for diagnosis or therapy of a disorder affecting the CNS for the preparation of a medicine capable of passing through the hemato-encephalic barrier to be used for diagnosis or therapy of a disorder localized in the CNS, the said peptide satisfying the following formula:
RRLSYSRRRF (Syn B3).
